Europäisches Patentamt

European Patent Office

Office européen des brevets

(19)

(11) Veröffentlichungsnummer: **0 265 742**

**A1**

(12) # EUROPÄISCHE PATENTANMELDUNG

(21) Anmeldenummer: 87114764.1

(51) Int. Cl.4: **A61B 17/22**

(22) Anmeldetag: 09.10.87

(30) Priorität: 23.10.86 DE 3636093

(43) Veröffentlichungstag der Anmeldung:
04.05.88 Patentblatt 88/18

(84) Benannte Vertragsstaaten:
**DE FR GB NL**

(71) Anmelder: **Siemens Aktiengesellschaft Berlin und München**
**Wittelsbacherplatz 2**
**D-8000 München 2(DE)**

(72) Erfinder: **Naser, Georg, Dipl.-Ing. (FH)**
**Karlstrasse 10**
**D-8502 Zirndorf(DE)**
Erfinder: **Reichenberger, Helmut, Dr.**
**Dipl.-Phys.**
**Begonienstrasse 28**
**D-8501 Eckental(DE)**

(54) **Lithotripter mit Ortungsvorrichtung.**

(57) Der Lithotripter (1) zur Behandlung von Konkrementen (K) ist mit einer Fokussierungseinrichtung (25) ausgerüstet. Er umfaßt weiter ein Gehäuse (3, 5) mit Ankoppelmembran (9) zum Austritt von Stoßwellen. In fester gegenseitiger räumlicher Zuordnung sind eine erste und eine zweite Ultraschall-Sende-und Empfangsvorrichtung (33, 35) angeordnet. Die beiden Vorrichtungen (33, 35) sind als Sektor-Scanner ausgebildet, deren Abtastebenen (E1, E2) einen Winkel von 90° miteinander bilden und durch die Zentralachse Z des Lithotripters (1) verlaufen. Der erste Sektor-Scanner (33) ist um die Zentralachse (Z) drehbar, und der zweite Sektor-Scanner (35) ist um eine senkrecht zur Zentralachse (Z) verlaufende Schwenkachse (21), die in der Abtastebene (E2) des zweiten Sektor-Scanners liegt, - schwenkbar. Die Fokussierungseinrichtung (25) ist entlang der Zentralachse (Z) verschiebbar.

Vorteil des Lithotripters ist die Möglichkeit der räumlichen Ortung des Konkrements (K) ohne Veränderung der Lage der Ankoppelmembran (9) in Relation zum Patienten (11). Diese Ortung kann auch bei komplizierten Konkrementlagen, wie sie insbesondere bei Gallensteinen auftreten, durchgeführt werden.

FIG 1

## Lithotripter mit Ortungsvorrichtung

Die Erfindung betrifft einen Lithotripter mit einer Fokussierungseinrichtung für einen entlang einer Zentralachse emittierten Stoßwellenimpuls, wobei an der Fokussierungseinrichtung in fester räumlicher Zuordnung eine erste und eine zweite Ultraschall-Sende und Empfangsvorrichtung angeordnet sind.

Ein derartiger Lithotripter ist beispielsweise aus der DE-OS 27 22 252 bekannt. Dort ist als Fokussierungseinrichtung ein Reflektor in Form eines Ellipsoids vorgesehen; an dessen Gehäusewand sind zwei Ultraschallwandler befestigt. Die Ultraschallwandler sind so justiert, daß sie den zweiten Brennpunkt des Ellipsoids unter einem Winkel von ca. 30° schneiden. Das Konkrement wird nach dem A-Bild-Verfahren identifiziert. Im dort beschriebenen Ausführungsbeispiel ist der Patient in einem Wasserbad angeordnet, so daß ein Verschieben des Lithotripters keine Probleme bei der Ankopplung der Stoßwellen an den Patienten mitsichbringt.

Anders sieht es aus, wenn bei einer sogenannten "trockenen Ankopplung" (vergl. DE-OS 33 28 051) der Lithotripter über eine Membran an den Patienten angekoppelt wird. Dann nämlich ist darauf zu achten, daß beim Ortungsvorgang die Ankoppelmembran möglichst unverändert am Patienten anliegt. Außerdem sollten bei einem Lithotripter keine Schwierigkeiten auftreten beim Orten von Konkrementen in komplizierten Lagen, wie sie sich z.B. in besonderem Maße bei Gallensteinen ergeben können.

Aus der DE-OS 34 27 001 ist eine Ortungs-und Positionierungseinrichtung bekannt, die mit einem an einer kardanischen Aufhängung geführten Ultraschallschwinger arbeitet. Eine dreidimensionale, d.h. räumliche Ortung ist hier nur mit erheblichem apparativen Aufwand möglich.

Aufgabe der Erfindung ist es daher, einen Lithotripter der eingangs genannten Art so auszubilden, daß auch komplizierte Konkrementlagen sicher räumlich geortet werden können, ohne daß der Zustand und die Lage der Ankoppelmembran am Patienten verändert werden müssen.

Diese Aufgabe wird erfindungsgemäß dadurch gelöst, daß beide Ultraschall-Sende-und Empfangsvorrichtungen als Sektor-Scanner ausgebildet sind, deren Abtastebenen einen vorgegebenen Winkel miteinander bilden und durch die Zentralachse verlaufen, daß die erste Ultraschall-Sende-und Empfangsvorrichtung unter Mitführung der zweiten Vorrichtung (und ggf. der Fokussierungseinrichtung) um die Zentralachse drehbar ist, und daß die Fokussierungseinrichtung unter Mitführung beider Ultraschall-Sende-und Empfangsvorrichtungen entlang der Zentralachse verschiebbar ist.

Besondere Vorteile liegen in der leichten Handhabbarkeit der Vorrichtung bei der Ortung eines Konkrements, insbesondere eines Gallensteins.

Weitere Vorteile und Ausgestaltungen der Erfindung ergeben sich aus der Beschreibung bevorzugter Ausführungsbeispiele anhand der Figuren. Es zeigen:

Figur 1 einen Lithotripter mit einer Fokussierungseinrichtung und zwei Ultraschall-Sektor-Scannern,

Figur 2 das In-Deckung-Bringen einer ersten Abtastebene E1 mit dem Konkrement K,

Figur 3 einen Querschnitt einer Ausgangslage bei der Konkrementortung,

Figur 4 einen Querschnitt der Fokussierungseinrichtung mit Sektor-Scannern im geschwenkten Zustand und

Figur 5 eine bevorzugte Triggereinrichtung.

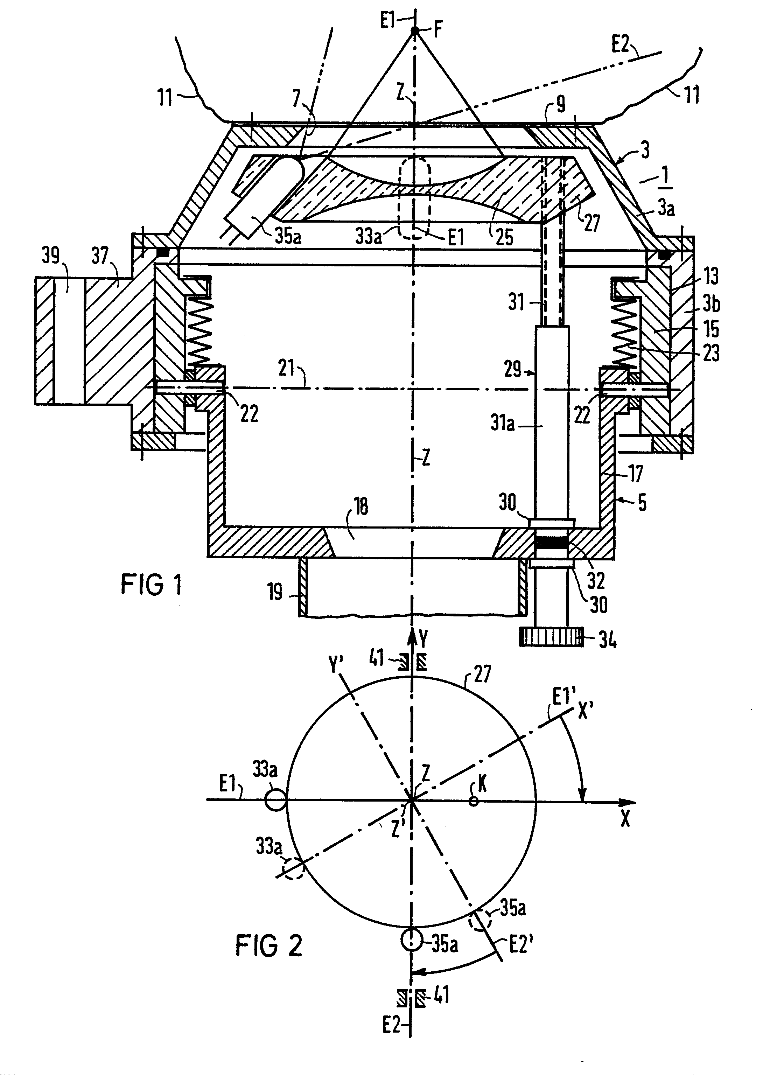

In Figur 1 ist ein Lithotripter generell mit 1 bezeichnet. Er umfaßt ein erstes und ein zweites Teilgehäuse 3 bzw. 5, die im wesentlichen rotationssymmetrisch ausgeführt sind. Im oberen Teil 3a des ersten Teilgehäuses 3 ist eine zentrale Aussparung 7 vorgesehen, die von einer Ankoppelmembran 9 überspannt ist. Durch die Aussparung 7 treten die mit dem Lithotripter 1 erzeugten Stoßwellenimpulse über die Ankoppelmembran 9 in den zu behandelnden Patienten 11 ein. Der untere, im wesentlichen ringförmige Teil 3b des ersten Teilgehäuses 3 ist über ein Gleitlager 13 drehbar mit dem zweiten Teilgehäuse 5 verbunden.

Das zweite Teilgehäuse 5 umfaßt einen nicht-schwenkbaren oberen Teil 15, der ringförmig ausgebildet und dessen Außenfläche vom Teil 3b eingefaßt ist, und einen schwenkbaren Topfteil 17, an dessen Boden unterhalb einer Öffnung 18 eine Stoßwellenquelle 19 befestigt ist. Eine solche Stoßwellenquelle 19 kann bevorzugt ein Stoßwellenrohr sein, wie es in der DE-OS 33 28 051 näher beschrieben ist. Die Teile 3b und 15 bilden miteinander das Dreh-Gleitlager 13.

Der schwenkbare Topfteil 17 ist um eine in der Papierebene liegende Schwenkachse 21, z.B. in Form zweier Zapfen 22, zu verschwenken oder zu verkippen. Die genaue Anordnung der Schwenkachse 21 wird weiter unten näher beschrieben. Um Wasserdichtigkeit des Lithotriptergehäuses 3, 5 zu erreichen, sind der schwenkbare Topfteil 17 und der nicht-schwenkbare Teil 15 über einen innen liegenden Balg 23 miteinander verbunden. Bei einem Schwenken des Topfteils 17 wird eine Hälfte des Balgs 23 zusammengedrückt und die andere Hälfte auseinandergezogen. Es ist eine Füllung des Gehäuses 3, 5 mit einem Dieleketrikum, wie z.B. Wasser, aus Gründen der Stoßwellenfortpflanzung erforderlich.

Die Stoßwellenquelle 19 weist eine Zentralachse Z auf, die zusammenfällt mit der Zentralachse einer im Gehäuse 3, 5 nahe der Membran 9 untergebrachten Fokussierungseinrichtung 25. Die Fokussierungseinrichtung 25 ist im gezeigten Ausführungsbeispiel eine bikonkave Linse. Die Sammellinse ist zentral zur Zentralachse Z angeordnet und über ein Verschiebemittel 29 in Richtung der Zentralachse Z verschiebbar. Dazu umfaßt das Verschiebemittel 29 beispielsweise drei jeweils um einen Winkel von 120° versetzte Drehstangen oder Drehgewinde 31. Im oberen Teil der Drehstangen 31 sind Gewinde eingeschnitten. Der untere, gewindefreie Teil 31a der Drehstangen 31 ist jeweils im Boden des Topfteils 17 drehbeweglich gelagert. Je zwei Sichtungsringe 30 halten jede Drehstange 31 axial bezüglich des Topfes 17 fest. Zur Abdichtung befindet sich in jedem Teil 31a zwischen den Sicherungsringen 30 in einer Nut ein O-Ring 32.

Die Drehgewinde 31 greifen randseitig in Innengewinde in der Linse oder der Fokussierungseinrichtung 25 ein. Ein gleichmäßiges Drehen der drei Drehgewinde 31 verschiebt die Linse 25 entlang der Zentralachse Z, also parallel zur Stoßwellen-Abstrahlfläche. Zu dieser linearen Verschiebung befindet sich auf dem Ende des gewindefreien Teils 31a ein Zahnrad 34. Alle drei Zahnräder 34 werden durch einen gemeinsamen Zahnriemen (nicht gezeigt) von einem Motor (nicht gezeigt) angetrieben.

Die Linse 25 weist eine Fokuspunkt F auf, der somit beim Verschieben auf der Zentralachse Z verbleibt.

Am Rand 27 der Linse 25, und zwar um einen fest vorgegebenen Winkel Alpha bezüglich der Achse Z gegeneinander versetzt, sind die Köpfe von zwei Ultraschall-Sende-und Empfangsvorrichtungen 33 bzw. 35 angeordnet, die als Sektor-Scanner ausgebildet sind. Im gezeigten Ausführungsbeispiel beträgt der Winkel Alpha 90°. Die erste Ultraschall-Sende-und Empfangsvorrichtung 33 ist gestrichelt dargestellt, da sie sich bezüglich des gezeigten Querschnitts vor der Betrachtungsebene befindet. Die Abtastebe ne E1 der ersten Ultraschall-Sende-und Empfangsvorrichtung 33 liegt senkrecht zur Papierebene und geht durch die Zentralachse Z.

Die zweite Abtastebene E2, die von dem zweiten als Ultraschall-Sende-und Empfangsvorrichtung 35 dienenden Sektor-Scanner erzeugt wird, leigt in der Papierebene. Sie ist mit Linien aus abwechselnd einem Strich und drei Punkten markiert. Auch die zweite Abtastebene E2 geht zwangsläufig durch die Zentralachse Z. Diese geometrischen Gegebenheiten (Abtastebenen E1, E2 senkrecht aufeinander und beide durch die Zentralachse Z der Fokussierungseinrichtung 25 verlaufend) sind fest vorgegeben. Die Ultraschallköpfe 33a, 35a sind dabei auf den Fokuspunkt F zu geneigt, in der Peripherie oder am Rand 27 der Linse 25 untergebracht und somit mit dieser fest verbunden.

Am Teil 3b des ersten Teilgehäuses 3 sind drei oder vier Verstärkungen oder Ausbuchtungen 37 vorgesehen (nur eine ist gezeigt), die jeweils eine Bohrung 39 parallel zur Zentralachse Z enthalten. Durch die Bohrungen 39 kann ein Gestänge verlaufen, mit dessen Hilfe der Lithotripter 1 an den Patienten 11 herangeführt und wieder abgenommen werden kann.

Die folgenden Figuren 2 bis 5 dienen der Veranschaulichung des Ortungsvorgangs, wie er mit dem unter Figur 1 beschriebenen Lithotripter 1 möglich ist. Figur 2 zeigt dabei eine schematische Aufsicht auf die wirksame Fokussierungseinrichtung 27 sowie auf den ersten Sektor-Scanner 33 mit zugeordneter erster Abtastebene E1 (Ebene X-Z) und den zweiten Sektor-Scanner 35 mit zugeordneter zweiter Abtastebene E2 (Ebene Y-Z). Zur Veranschaulichung, daß die zweite Abtastebene E2 um die Schwenkachse 21 (vergl. Figur 1) - schwenkbar ist, die die Y-Achse mit einem symbolischen Schwenkachslager 41 versehen. Die sektorförmigen Abtastebenen E1, E2 können auf den Bildschirmen der Vorrichtungen 33 bzw. 35 betrachtet werden.

In Figur 2 ist gestrichelt eine Konstellation der Achsen X', Y', Z' -wobei hier Z' mit Z identisch ist - des Lithotripters 1 zu dem Konkrement K im Inneren des Patienten 11 gezeigt, wie sie sich zunächst zufällig ergibt, wenn der Lithotripter 1 beim ersten Ansetzen noch ungerichtet an den Patienten 11 angekoppelt wird. Das Konkrement K liegt an irgendeiner Stelle zwischen den noch nicht ausgerichteten Abtastebenen E1' und E2'.

Als erster Schritt zur genauen Konkrementortung und Lithotripterjustierung wird das zweite Teilgehäuse 5 mit Hilfe des Gleitlagers 13 so lange um die Zentralachse Z unter Mitnahme beider Vorrichtungen 33, 35 gedreht, bis das Konkrement K in der ersten Abtastebene E1 des ersten Ultraschall-Scanners 33 auftaucht. Dieses entspricht den durchgezogenen Achsen X, Y in Figur 2. Die Vorrichtungen 33, 35 sind hier der Deutlichkeit wegen außerhalb der Linse 27 dargestellt. Vorliegend wurde die gesamte Anordnung 25, 33, 35 gedreht.

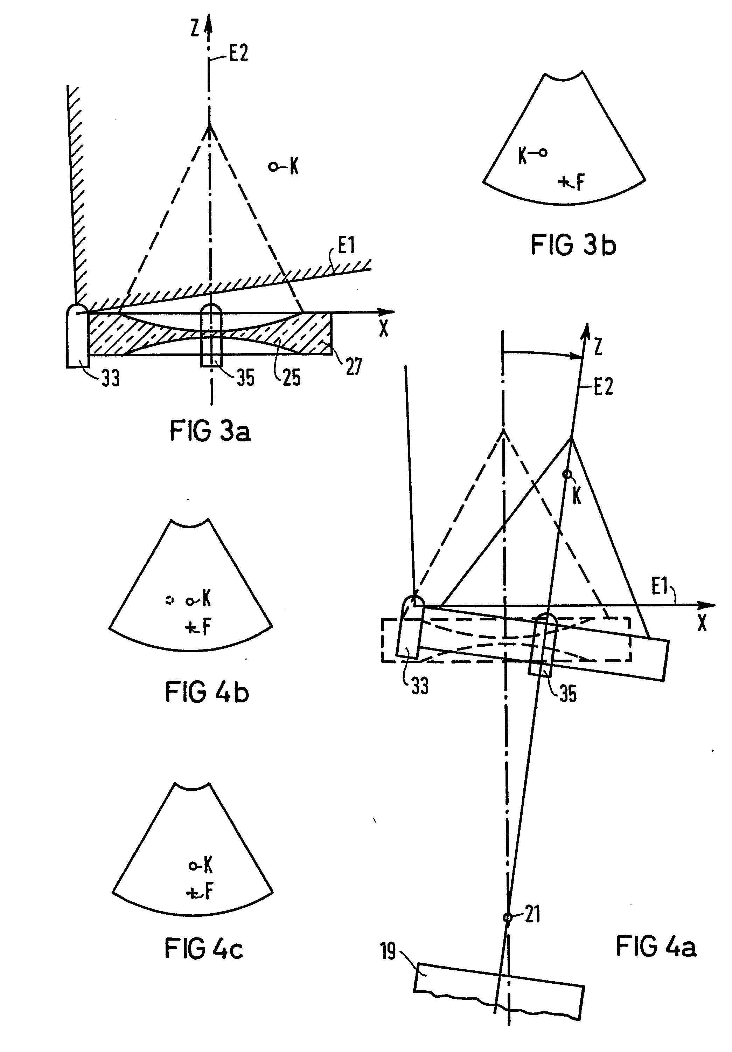

Figur 3a zeigt den zu dieser Position zugehörigen Querschnitt durch den Lithotripter 1. Die in der Papierebene liegende Abtastebene E1 (X-Z-Ebene) des ersten Sektor-Scanners 33 hat das Konkrement K geortet. Es ergibt sich das als Beibld 3b gezeigte Bildschirm-Bild von Konkrement K und eingeblendetem Fokus F. Die Abtastebene E2 (senkrecht zur Papierebene durch Z) geht noch am Konkrement K vorbei. Der Bildschirm des zweiten Sektor-Scanners 35 zeigt also noch nicht das Konkrement K.

In Figur 4a ist der Inhalt von Figur 3 gestrichelt als Ausgangslage für den nächsten Ortungsschritt dargestellt. Die gesamte Anordnung von Stoßwellenquelle 19 und Fokussierungseinrichtung 25 nebst fest zugeordneten Ultraschall-Sende-und Empfangsvorrichtungen 33 und 35 wird um die (senkrecht zur Papierebene stehende) Schwenkachse 21 solange gekippt, bis das Konkrement K auf dem Bildschirm des zweiten Sektor-Scanners 35 auftaucht. Die Schwenkachse 21 ist dabei so ausgerichtet, daß das Konkrement K gleichzeitig im Abtastbereich des ersten Ultraschall-Scanners 33 verbleibt. Das bedeutet, daß die Schwenkachse 21 sowohl senkrecht zur Zentralachse Z angeordnet sein als auch in der nach hinten verlängert gedachten Abtastebene E2 des zweiten Ultraschall-Scanners 35 liegen muß.

Die beiden zu Figur 4a gezeigten Beibilder 4b, 4c zeigen das Ultraschall-Bild des ersten Sektor-Scanners 33 vor (in gestrichelter Darstellung) und nach dem Schwenkvorgang bzw. das Ultraschall-Bild des zweiten Sektor-Scanners 35 nach dem Schwenkvorgang. Vor dem Schwenkvorgang war das Konkrement K auf diesem Ultraschall-Bild 4c noch nicht zu sehen. Nach dem zweiten Positionierschritt ist der Lithotripter 1 so ausgerichtet, daß das Konkrement K auf der Zentralachse Z liegt. Es ist also auf dem zweiten Bildschirm zu sehen.

Die Tiefenlage des Konkrements K auf der Zentralachse Z muß allerdings noch nicht mit dem Fokus F der Fokussierungseinrichtung 25 zusammenfallen. Durch Verdrehen der Drehgewinde 31 des Verschiebemittels 29 wird nun im dritten Schritt der Fokuspunkt F in das Konkrement K verlegt. Damit ist der Fokussierungsvorgang abgeschlossen, und der erste Stoßwellenimpuls kann ausgelöst werden.

Vorteil des Lithotripters 1 ist die Verschiebbarkeit des Fokuspunkts F, ohne daß die Lage der Ankoppelmembran 9 in Relation zum Patienten 11 verändert werden muß. Durch die Möglichkeit zum Verdrehen und zum Schwenken der Abtastebenen E1 und E2 der Ultraschall-Scanner 33 bzw. 35 wird die Möglichkeit geschaffen, auch bei komplizierten Steinlagen, wie sie insbesondere bei Gallensteinen auftreten, eine sichere Positionierung vorzunehmen. Die Zuverlässigkeit der Behandlung ist somit gewährleistet.

Statt mit einem Verstellmechanismus, der die vorangehend beschriebenen Funktionen "Drehung um die Z-Achse" und die nachfolgende "Schwenkung" durchführt, kann desselbe Ergebnis auch mit einer Anordnung erreicht werden, mit der eine zweimalige Schwenkung um Achsen parallel zur X-und zur Y-Achse möglich ist. Alternativ dazu kann desselbe Ergebnis auch mit einer Anordnung erzielt werden, die eine zweimalige Verschiebung in X-und Y-Richtung ermöglicht.

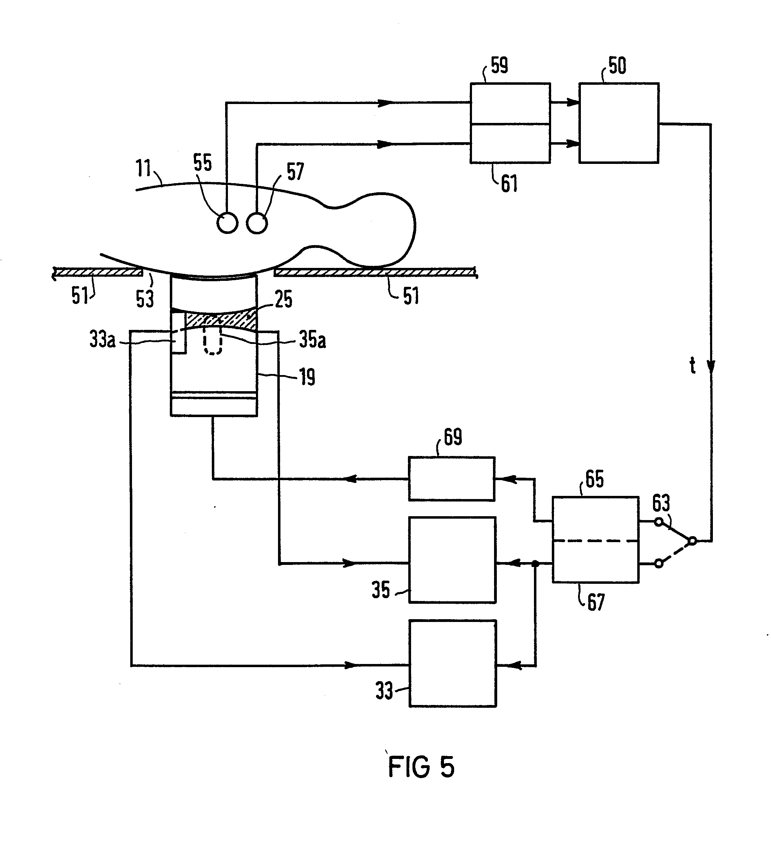

In Figur 5 ist eine besonders wichtige weitere Ausgestaltung dargestellt. Hier wird davon ausgegangen, daß die Bewegung eines Konkrements, die insbesondere durch die Atemtätigkeit verursacht sein kann, zu einem fortwährenden Auftreten und Verschwinden in den beiden Ultraschall-Bildern führen kann. Entsprechendes kann - in geringerem Maße - auch für die Herztätigkeit gelten. Daher ist es vorteilhaft, wenn die Aufnahme der Ultraschall-Bilder jeweils in der Atemlage und/oder EKG-Position erfolgt, in der auch die Stoßwelle ausgelöst werden soll. Für die Beurteilung durch den behandelnden Arzt ist es ebenso vorteilhaft, wenn nur diese Ultraschall-Bilder dargestellt werden.

Nach Figur 5 ist für diesen Zweck eine gemeinsame Triggereinrichtung 50 vorgesehen, die sowohl bei der Aufnahme der Ultraschall-Bilder aus auch bei der Auslösung der Stoßwellen herangezogen wird. Zu diesem Zweck sind am Patienten 11, der auf einer Patienten-Lagerungsplatte 51 mit Druchtrittsöffnung 53 für die Stoßwellen liegt, Abnehmer oder Sensoren 55 und 57 für die Atmung bzw. für die Herztätigkeit (EKG) angeordnet. Die Ausgangssignale dieser Sensoren 55, 57 sind entsprechenden (an sich bekannten) Auswertegeräten 59 bzw. 61 zugeführt, deren Ausgangssignale vorliegend gemeinsam der Triggereinrichtung 50 zugeleitet sind. Für viele Zwecke ist es ausreichend, lediglich eine Einrichtung 55, 59 zur Atmungsüberwachun vorzusehen und auf die Herztriggerung zu verzichten.

Die Triggereinrichtung 50 bildet aus den zugeführten Signalen in bekannter Weise ein Triggersignal t, das über einen Wechselschalter 63 entweder einem Auslösegerät 65 für die Stoßwellen oder einem Aufnahme-Startgerät 67 für die Ultraschall-Bilder zugeführt wird. In der gezeigten Schaltstellung wird mit dem Triggersignal t die zur Stoßwellenquelle 19 gehörende Stoßwel leneinrichtung 69 angesteuert. Die Stoßwellenquelle 19 ist vorliegend als (an sich bekanntes) Stoßwellenrohr dargestellt.

In der anderen Stellung des Schalters 63 beaufschlagt das Triggersignal t das Aufnahme-Startgerät 67 für die Ultraschall-Bilder der beiden Ultraschall-Scanner 33 und 35. Die von den Köpfen 33a bzw. 35a aufgenommene Echosignale werden mit Hilfe der Einrichtungen 33 bzw. 35 auf (nicht gezeigten) Bildschirmen dargestellt. Nach Figur 5 erfolgt also die Aufnahme der beiden Ultraschall-Bilder jeweils in derjenigen Atemlage, in der auch nachfolgend die Stoßwelle ausgelöst wird. Dies kann - wie ausgeführt - auch für die Phasenlage die Herztätigkeit gelten.

## Ansprüche

1. Lithotripter mit einer Fokussierungseinrichtung für einen entlang einer Zentralachse emittierten Stoßwellenimpuls, wobei an der Fokussierungseinrichtung in fester räumlicher Zuordnung eine erste und eine zweite Ultraschall-Sende-und Empfangsvorrichtung angeordnet sind, **dadurch gekennzeichnet,** daß beide Ultraschall-Sende-und Empfangsvorrichtung (33, 35) als Sektor-Scanner ausgebildet sind, deren Abtastebenen (E1, E2) einen vorgegebenen Winkel (Alpha) miteinander bilden und durch die Zentralachse (Z) verlaufen, daß die erste Ultraschall-Sende-und Empfangsvorrichtung (33) unter Mitführung der zweiten Vorrichtung (35) um die Zentralachse (Z) drehbar ist, und daß die Fokussierungseinrichtung (25) unter Mitführung beider Ultraschall-Sende-und Empfangsvorrichtungen (33, 35) entlang der Zentralachse (Z) durch Verschiebemittel verschiebbar ist.

2. Lithotripter nach Anspruch 1, **dadurch gekennzeichnet,** daß die zweite Ultraschall-Sende-und Empfangsvorrichtung (35) unter Mitführung der ersten Vorrichtung (33) um eine senkrecht zur Zentralachse (Z) verlaufende Schwenkachse (21), die in der Abtastebene (E2) der zweiten Vorrichtung (35) liegt, verlaufenden Achse (X, Y) verschiebbar ist.

3. Lithotripter nach Anspruch 1 oder 2, **dadurch gekennzeichnet,** daß der Winkel (Alpha) 90° beträgt.

4. Lithotripter nach Anspruch 1, 2 oder 3, **dadurch gekennzeichnet,** daß die Fokussierungseinrichtung (25) eine Linse ist, an der die Ultraschall-Sende-und Empfangsvorrichtungen (33, 35) befestigt sind.

5. Lithotripter nach einem der Ansprüche 1 bis 4, **dadurch gekennzeichnet,** daß die Fokussierungseinrichtung (25) vor einer Ankoppelmembran (9) und um die Zentralachse (Z) drehbar angeordnet ist.

6. Lithotripter nach Anspruch 5, **dadurch gekennzeichnet,** daß sein Gehäuse zwei Teilgehäuse (3, 5) umfaßt, wobei das erste Teilgehäuse (3) die Ankoppelmembran (9) umfaßt und das zweite Teilgehäuse (5) die Stoßwellenquelle (19) beinhaltet.

7. Lithotripter nach Anspruch 6, **dadurch gekennzeichnet,** daß beide Teilgehäuse (3, 5) mittels eines zylindrischen Gleitlagers (13) gegeneinander drehbar gelagert sind.

8. Lithotripter nach Anspruch 6 oder 7, **dadurch gekennzeichnet,** daß das zweite Teilgehäuse (5) einen nicht-schwenkbaren Teil (15) und einen um die Schwenkachse (21) schwenkbar gelagerten Topfteil (17) umfaßt, an dessen Boden die Stoßwellenquelle (19) angeordnet und der über einen Balg (23) mit dem nicht-schwenkbaren Teil (15) verbunden ist.

9. Lithotripter nach einem der Ansprüche 6 bis 8, **dadurch gekennzeichnet,** daß der von beiden Teilgehäusen (3, 5) umschlossene Raum mit einem flüssigen Dielektrikum, wie z.B. Wasser, gefüllt ist.

10. Lithotriptor nach einem der Ansprüche 8 oder 9, **dadurch gekennzeichnet,** daß die Fokussierungseinrichtung (25) mit dem schwenkbar gelagerten Topfteil (17) über parallel zur Zentralachse (Z) im Gehäuse angeordnete Verschiebemittel (29) verbunden ist.

11. Lithotripter nach Anspruch 10, **dadurch gekennzeichnet,** daß die Verschiebemittel (29) ein Drehgewinde (31) umfassen.

12. Lithotripter nach einem der Ansprüche 1 bis 11, **gekennzeichnet durch** eine phasengleiche Ultraschall-Bildaufnahme und Stoßwellenauslösung.

13. Lithotripter nach Anspruch 12, **dadurch gekennzeichnet,** daß die Triggerung der Ultraschall-Bildaufnahme und der Stoßwellenauslösung die Atmung und/oder das EKG vorgesehen sind/ist.

14. Lithotripter nach Anspruch 13, **dadurch gekennzeichnet,** daß eine gemeinsame Triggereinrichtung (50) vorgesehen ist, die sowohl zur Ultraschall-Bildaufnahme als auch zur Stoßwellenauslösung in jeweils gleicher Atemlage vorgesehen ist.

15. Lithotripter nach einem der Ansprüche 1 bis 14, **dadurch gekennzeichnet,** daß beide Ultraschall-Sende-und Empfangsvorrichtungen (33, 35) am Rand (27) der Fokussierungseinrichtung (25) angeordnet sind.

86 P 3397    E

FIG 1

FIG 2

FIG 3b

FIG 3a

FIG 4b

FIG 4c

FIG 4a

FIG 5

<table>
<tr><td colspan="4"><b>EUROPÄISCHER RECHERCHENBERICHT</b></td></tr>
</table>

**Europäisches Patentamt**

# EUROPÄISCHER RECHERCHENBERICHT

Nummer der Anmeldung

EP 87 11 4764

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| Y | EP-A-0 131 653 (DE OUDE DELFT) * Figuren 2,3,5; Seite 4, Zeile 1 - Seite 5, Zeile 7; Seite 6, Zeilen 16-27 * | 1-3,5-7 ,9-11 | A 61 B 17/22 |
| A | | 8 | |
| | --- | | |
| Y | EP-A-0 081 639 (DORNIER) * Figur 1; Seite 3, Zeilen 4-13; Seite 3, Zeile 26 - Seite 4, Zeile 4; Seite 5, Zeilen 1-5,15-23; Anspruch 1 * | 1-3,5-7 ,9-11 | |
| | --- | | |
| A | EP-A-0 081 051 (DORNIER) * Figur 2; Seite 2, Zeilen 1-17; Ansprüche 1-4 * | 12-14 | |
| | --- | | |
| A,D | DE-A-2 722 252 (DORNIER) * Figur 1; Seite 12, Zeile 21 - Seite 13, Zeile 5 * | 15 | |
| | ----- | | |

### RECHERCHIERTE SACHGEBIETE (Int. Cl.4)

A 61 B
G 01 S

Der vorliegende Recherchenbericht wurde für alle Patentansprüche erstellt

| Recherchenort | Abschlußdatum der Recherche | Prüfer |
|---|---|---|
| DEN HAAG | 20-01-1988 | SEDY, R. |

EPO FORM 1503 03.82 (P0403)